# FASCICULE DE BREVET EUROPEEN

(11) **EP 3 125 859 B1**
(45) Date de publication et mention de la délivrance du brevet: **12.06.2019**
(21) Numéro de dépôt: 15718539.8
(22) Date de dépôt: 02.04.2015
(51) Int. Cl.: A61K 8/40, A61K 8/87, A61L 26/00, C08K 5/32, C08L 75/04

(54) **COMPOSITION FILMOGENE**
FILMBILDENDE ZUSAMMENSETZUNG
FILM-FORMING COMPOSITION

(30) Priorité: 03.04.2014 FR 1452947
(43) Date de publication de la demande: 08.02.2017
(73) Titulaire: URGO RECHERCHE INNOVATION ET DEVELOPPEMENT, 21300 Chenôve (FR)
(72) Inventeur: RAVENET, Anne-Laure, F-71380 Alleriot (FR); BOUVIER, Claire, F-21000 Dijon (FR); DERAIN, Nathalie, F-21370 Prenois (FR)
(74) Mandataire: Cabinet Plasseraud
(86) Numéro de dépôt international: PCT/FR2015/050856
(87) Numéro de publication internationale: WO 2015/150705

(56) Documents cités:
- EP-A1- 0 969 313
- EP-A1- 1 057 846
- EP-A2- 2 606 934
- EP-B1- 1 893 248
- WO-A1-2005/099782
- WO-A1-2009/070736
- WO-A1-2012/175881

## Description

La présente invention a pour objet une composition liquide filmogène comprenant au moins un polymère filmogène dérivé du polyuréthane, au moins un épaississant et au moins un acide hydroxamique. L'invention a également pour objet un procédé d'obtention d'un film à partir de ladite composition liquide. L'invention se rapporte également à l'utilisation d'un acide hydroxamique pour stabiliser la viscosité d'une telle composition.

Les compositions filmogènes sont destinées à être appliquées, avec ou sans applicateur, sur des tissus tels que la peau, les phanères (les ongles ou les cheveux), les muqueuses. Elles peuvent également être utilisées comme pansement liquide, et sont alors appliquées sur des plaies, des lésions, des cicatrices, des tissus brûlés et/ou affections cutanées. Elles sont généralement liquides à l'application et contiennent classiquement un polymère filmogène dissous dans un solvant volatil, typiquement de l'eau ou un alcool. L'évaporation du solvant permet alors la formation d'un film solide protecteur.

Les compositions filmogènes doivent présenter une texture contrôlée, suffisamment fluide pour pouvoir être appliquées sur la zone à traiter et former un film uniforme, mais aussi suffisamment épaisses pour rester sur la zone à traiter le temps du séchage et former un film présentant une épaisseur appropriée.

Il est donc souvent souhaitable d'introduire dans ces compositions des agents épaississants capables de texturer la formulation, sans toutefois la rendre trop solide. Cependant, la viscosité optimale obtenue lors de la formulation des compositions a tendance à diminuer au cours du temps, notamment lors du stockage des compositions qui peut durer plusieurs mois. Les compositions deviennent alors trop fluides, voire déphasent, ce qui rend le produit fini inutilisable.

Il existe donc un besoin pour de nouvelles compositions filmogènes non cytotoxiques, capables de former in situ et rapidement des films solides résistants à la rupture, présentant une texture optimale permettant leur étalement facile et uniforme sur la zone à traiter, et présentant une viscosité stable dans le temps.

La Demanderesse a trouvé, de manière surprenante, qu'il était possible de former de tels films sur tissus tels que la peau, les phanères ou les muqueuses, présentant une viscosité stable dans le temps, ainsi que d'excellentes propriétés de résistance à la rupture, d'adhérence, et d'élasticité, au moyen d'une composition liquide particulière.

L'invention a ainsi pour objet, selon un premier aspect, une composition liquide comprenant au moins un polymère filmogène dérivé du polyuréthane présent en une teneur en matières sèches allant de 25 % à 60 % en poids, de préférence allant de 30 % à 55 % en poids, par rapport au poids total de la composition, au moins un épaississant et au moins un acide hydroxamique.

Les compositions filmogènes à base de polyuréthane sont connues de l'homme du métier. Le brevet EP 1 893 248 décrit par exemple d'associer un polyuréthane et un polyacrylate dans une composition filmogène, et d'appliquer ces compositions sur la peau pour former un film protecteur. Le film obtenu présente notamment l'avantage d'être résistant aux rayonnements gamma mis en oeuvre pour la stérilisation, ce qui permet de l'utiliser dans un grand nombre d'applications médicales. Toutefois, ce document n'enseigne pas les problèmes liés à la stabilité au stockage ce type de composition lorsqu'elles comprennent un agent épaississant, et ne propose par conséquent aucune solution pour améliorer la stabilité de telles compositions.

Selon un second aspect, l'invention a également pour objet un procédé d'obtention d'un film à partir de la composition liquide précédemment décrite, caractérisé en ce qu'on applique ladite composition sur les tissus tels que la peau, les phanères ou les muqueuses, de manière à former un film uniforme.

Selon un dernier aspect, l'invention se rapporte également à l'utilisation d'un acide hydroxamique pour stabiliser la viscosité d'une telle composition.

En particulier, la composition liquide selon l'invention est physiologiquement acceptable, c'est à dire non toxique et susceptible d'être appliquée sur les tissus tels que la peau, les plaies, les phanères ou les muqueuses d'êtres humains ou d'animaux et permet la formation d'un film biocompatible.

On entend par composition liquide, au sens de la présente demande, une composition qui s'écoule sous son propre poids.

### Polymère filmogène dérivé du polyuréthane

La composition liquide comprend au moins un polymère filmogène dérivé du polyuréthane.

Selon la présente invention, on entend par "polymère filmogène", un polymère apte à former à lui seul ou en présence d'un agent auxiliaire de filmification, un film continu et adhérent sur un support, notamment sur la peau.

Dans le cadre de la présente invention le polymère filmogène est un polymère dérivé du polyuréthane, se présentant de préférence sous la forme d'une dispersion aqueuse de polyuréthane.

De manière préférentielle, le polymère dérivé du polyuréthane est un Polyuréthane-32 tel que celui commercialisé sous la dénomination Baycusan C1003 par la société Bayer.

Le polyuréthane est préférentiellement un produit de la réaction de :
A) un prépolymère de formule : 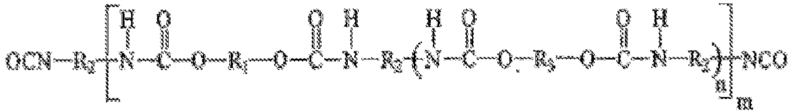 où
   R₁ représente un radical radical polyhydroxyle, de préférence dihydroxyle,
   R₂ représente un radical hydrocarboné issu d'un polyisocyanate aliphatique ou cycloaliphatique,
   R₃ représente un radical hydrocarboné issu d'un diol, éventuellement de faible poids moléculaire, éventuellement substitué par des groupes ioniques,
   n vaut 0 à 5, et
   m est> 1 ;
B) au moins un agent d'allongement de chaîne selon la formule :

   H₂N-R₄-NH₂

   où R₄ représente un radical alkylène ou oxyde d'alkylène non substitué par des groupes ioniques ou potentiellement ioniques ; et
C) au moins un agent d'allongement de chaîne de formule :

   R₅-NH-R₅'-NH₂

   où R₅ représente un radical alkylène substitué par des groupes ioniques ou potentiellement ioniques, et R₅' représente un radical alkyle, notamment en C₂-C₄.

### Prépolymère A)

### Radical R1

Des composés appropriés pour fournir le radical polyhydroxyle, de préférence dihydroxyle, R₁ sont les polyesterpolyols, de préférence les polyesterdiols, et leurs mélanges.

Ces composés sont ainsi avantageusement divalents, de préférence deux groupes hydroxy.

De tels composés peuvent présenter des poids moléculaires numériques moyens d'environ 700 à environ 16 000, et de préférence d'environ 750 à environ 5000.

Le(s) polyesterdiol(s) peuvent généralement être préparés à partir :
- d'acides dicarboxyliques ou polycarboxyliques aliphatiques, cycloaliphatiques ou aromatiques ou leurs anhydrides et
- d'alcools dihydriques tels que des diols choisis parmi les diols aliphatiques, alicycliques, aromatiques.

Les acides dicarboxyliques ou polycarboxyliques aliphatiques peuvent être choisis parmi : l'acide succinique, fumarique, glutarique, 2,2-diméthylglutarique, adipique, itaconique, pimélique, subérique, azélaïque, sébacique, maléique, malonique, 2,2- diméthylmalonique, nonanedicarboxylique, décanedicarboxylique, dodécanedioïque, 1,3-cyclohexanedicarboxylique, 1,4-cyclohexa-nedicarboxylique, 2,5-norborane dicarboxylique, diglycolique, thiodipropionique, 2,5-naphtalènedicarboxylique, 2,6- naphtalènedicarboxylique, phtalique, téréphtalique, isophtalique, oxanique, o-phtalique, tétrahydrophtalique, hexahydrophtalique ou trimellitique.

Les anhydrides d'acides peuvent être en particulier choisi parmi l'anhydride de l'acide o-phtalique, trimellitique ou succinique ou un mélange de ceux-ci.

De préférence l'acide dicarboxylique préféré est l'acide adipique.

Les alcools dihydriques peuvent être choisis parmi l'éthanediol, l'éthylène glycol, le diéthylèneglycol, le triéthylène glycol, le triméthylèneglycol, le tétraéthylèneglycol, le 1,2-propanediol, le dipropylèneglycol, le tripropylèneglycol, le tétrapropylèneglycol, le 1,3-propanediol, le 1,4-butanediol, le 1,3-butanediol, le 2,3-butanediol, le 1,5-pentanediol, le 1,6-hexanediol, le 2,2-diméthyl-1,3-propanediol, le 1,4-dihydroxycyclohexane, le 1,4-diméthylolcyclohexane, le cyclohexane diméthanol, le 1,8-octanediol, le 1,10-décanediol, le 1,12-dodécanediol, le neopentylglycol ou leurs mélanges.

Les composés dihydroxyliques cycloaliphatiques et/ou aromatiques conviennent bien entendu également comme alcool(s) dihydrique(s) pour la préparation de polyesterpolyol(s).

Les polyesterdiols peuvent aussi être choisis parmi les homopolymères ou copolymères de lactones, qui sont de préférence obtenus par des réactions d'addition de lactones ou des mélanges de lactones, tels que la butyrolactone, 1' ε-caprolactone et/ou la méthyl- ε-caprolactone avec les molécules initiatrices polyfonctionnelles, de préférence difonctionnelles appropriées, telles que par exemple les alcools dihydriques cités ci-dessus. Les polymères correspondants d'ε-caprolactone sont préférés.

Le radical polyesterpolyol R₁, de préférence polyester diol, peut être obtenu avantageusement par polycondensation d'acides dicarboxyliques, tel que l'acide adipique, avec des polyols, notamment des diols, tels que l'hexanediol, le neopentylglycol, et leurs mélanges.

### Radical R₂

Des polyisocyanates appropriés pour fournir le radical hydrocarboné R₂ comprennent les diisocyanates organiques présentant un poids moléculaire d'environ 112 à 1000, et de préférence d'environ 140 à 400.

Des diisocyanates préférés sont ceux représentés par la formule générale R₂(NCO)₂ indiquée ci-dessus, dans laquelle R₂ représente un groupe hydrocarboné aliphatique divalent comprenant 4 à 18 atomes de carbone, un groupe hydrocarboné cycloaliphatique divalent comprenant 5 à 15 atomes de carbone, un groupe hydrocarboné araliphatique divalent comprenant 7 à 15 atomes de carbone ou un groupe hydrocarboné aromatique divalent comprenant 6 à 15 atomes de carbone.

Des exemples de diisocyanates organiques qui conviennent comprennent le tétraméthylènediisocyanate, le 1,6-hexaméthylènediisocyanate, le dodécaméthylènediisocyanate, le cyclohexane-1,3-diisocyanate et le cyclohexane-1,4- di isocyanate, le 1-isocyanato-3-isocyanatométhyl-3,5,5-triméthylcyclohexane (isophoronediisocyanate ou IPDI), le bis-(4-isocyanatocyclohexyl)-méthane, le 1,3-bis(isocyanatométhyl)-cyclohexane et le 1,4-bis(isocyanatométhyl)-cyclohexane, le bis- (4-isocyanato-3-méthyl-cyclohexyl)-méthane. Selon un mode préféré de réalisation, des mélanges de diisocyanates peuvent être utilisés. Des diisocyanates préférés sont des diisocyanates aliphatiques et cycloaliphatiques.

On préfère en particulier le 1,6- hexaméthylènediisocyanate, l'isophoronediisocyanate et plus particulièrement leurs mélanges.

### Radical R₃

L'utilisation de diols, notamment de bas poids moléculaire, R₃ peut permettre une rigidification de la chaîne polymérique et est optionnelle.

L'expression "diols de bas poids moléculaire" signifie des diols présentant un poids moléculaire d'environ 62 à 700, de préférence de 62 à 200. Ils peuvent contenir des groupes aliphatiques, alicycliques ou aromatiques. Les composés préférés ne contiennent que des groupes aliphatiques.

Les diols utilisés présentent de préférence jusqu'à 20 atomes de carbone et peuvent être choisi parmi l'éthylèneglycol, le diéthylèneglycol, le propane-1,2-diol, le propane-1,3-diol, le butane-1,4-diol, le butylène-1,3-glycol, le néopentylglycol, le butyléthylpropanediol, le cyclohexanediol, le 1,4-cyclohexanediméthanol, l'hexane-1,6-diol, le bisphénol A (2,2- bis(4-hydroxyphényl)propane), le bisphénol A hydrogéné (2,2-bis(4- hydroxycyclohexyl)propane), et leurs mélanges. De préférence, R₃ est issu du butane-1,4-diol.

Eventuellement, les diols de bas poids moléculaire peuvent contenir des groupes ioniques ou potentiellement ioniques. Des diols de bas poids moléculaire appropriés contenant des groupes ioniques ou potentiellement ioniques sont ceux divulgués dans le brevet US 3412054. Des composés préférés comprennent l'acide diméthylolbutanoïque (DMBA), l'acide diméthylolpropionique (DMBA) et le caprolactonepolyesterdiol contenant carboxyle. Si des diols de bas poids moléculaire contenant des groupes ioniques ou potentiellement ioniques sont utilisés, ils sont de préférence utilisés en une quantité telle que <0,30 éq.mol. de COOH est présent par gramme de polyuréthane dans la dispersion de polyuréthane. De préférence, les diols de bas poids moléculaire contenant des groupes ioniques ou potentiellement ioniques ne sont pas utilisés.

La chaîne du prépolymère est allongée en utilisant deux classes d'agent d'allongement de chaîne B) et C).

Les composés B) de la première classe d'allongement de chaîne présentent la formule :

H₂N-R₄-NH₂

où R₄ représente un radical alkylène ou oxyde d'alkylène non substitué par des groupes ioniques ou potentiellement ioniques.

Ainsi, l'allongeur de chaîne peut être choisi parmi :
- Les alkylènediamines telles que l'hydrazine, l'éthylènediamine, la propylènediamine, la 1,4-butylènediamine et la pipérazine.
- Les diamines d'oxyde d'alkylène telles que le dipropylaminediéthylèneglycol (DPA-DEG disponible auprès de Tomah Products, Milton, WI), la 2-méthyl-1,5- pentanediamine (Dytec A de DuPont), l'hexanediamine, l'isophoronediamine, et la 4,4-méthylènedi(cyclohexylamine), et la série des DPA-étheramines disponibles chez Tomah Products, Milton, WI, comprenant le dipropylaminepropylèneglycol, le dipropylaminedipropylèneglycol, le dipropylaminetripropylèneglycol, le dipropylaminepoly(propylèneglycol), le dipropylaminéthylèneglycol, le dipropylaminepoly(éthylèneglycol), le dipropylamine-1,3-propanediol, le dipropylamine-2-méthyl-1,3-propanediol, le dipropylamine-1,4-butanediol, le dipropylamine-1,3-butanediol, le dipropylamine-1,6-hexanediol et le dipropylaminecyclohexane-1,4-diméthanol, et leurs mélanges.

De préférence, l'allongeur de chaîne B) est l'éthylènediamine.

Les composés C) de la seconde classe d'allongement de chaîne présentent la formule :

R₅-NH-R₅'-NH₂

où R₅ représente un radical alkylène substitué par des groupes ioniques ou potentiellement ioniques, et R₅' représente un radical alkyle, notamment en C₂-C₄.

Ces composés présentent un groupe ionique ou potentiellement ionique et deux groupes qui sont réactifs avec des groupes isocyanate.

Le groupe ionique ou le groupe potentiellement ionique peut être choisi dans le groupe constitué par les groupes d'ammonium tertiaire ou quaternaire, les groupes convertibles en un tel groupe, un groupe carboxyle, un groupe carboxylate, un groupe acide sulfonique et un groupe sulfonate. Ladite conversion au moins partielle des groupes convertibles en groupes de sel du type mentionné peut avoir lieu avant ou pendant le mélange avec l'eau. Des composés spécifiques comprennent les diaminosulfonates, tels que par exemple le sel sodique de l'acide N-(2-aminoéthyl)-2-aminopropanoique, le sel sodique de l'acide N-(2-aminoéthyl)-2-aminoéthanesulfonique ou sel sodique de l'acide N-(2-aminoéthyl)-3- aminoéthanesulfonique.

De préférence, R₅ représente un radical alkylène substitué par des groupes d'acide sulfonique ou sulfonate.

De préférence, le composé C) est le sel de sodium de l'acide N-(2-aminoéthyl)-3-aminoéthanesulfonique, répondant notamment à la formule

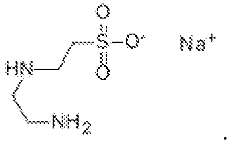

### Terminaisons de chaîne

Le polyuréthane selon l'invention peut également comprendre des composés qui sont situés dans chaque cas aux extrémités des chaînes et terminent lesdites chaînes. Ces terminaisons de chaîne peuvent dériver de composés présentant la formule 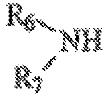 dans laquelle
R₆ représente un atome d'hydrogène ou un radical alkylène présentant éventuellement une extrémité hydroxyle et
R₇ représente un radical alkylène présentant éventuellement une extrémité hydroxyle.

Des composés appropriés comprennent les composés tels que les monoamines, en particulier les monoamines secondaires ou les monoalcools. Des exemples comprennent : la méthylamine, l'éthylamine, la propylamine, la butylamine, l'octylamine, la laurylamine, la stéarylamine, l'isononyloxypropylamine, la diméthylamine, la diéthylamine, la dipropylamine, la dibutylamine, la N-méthylaminopropylamine, la diéthyl(méthyl)aminopropylamine, la morpholine, la pipéridine, la diéthanolamine et des dérivés substitués appropriés de celle-ci, les amide-amines de diamines primaires et d'acides monocarboxyliques, les monocétimes de diamines primaires, les amines primaires/tertiaires telles que la N,N- diméthylamino-propylamine et analogues. Les alcools de terminaison de chaîne peuvent être choisis parmi les alcools en C₁-C₁₀, tels que le méthanol, le butanol, l'hexanol, l'alcool 2-éthylhexylique, l'alcool isodécylique, et leurs mélanges, les aminoalcools tels que l'aminométhylpropanol (AMP) conviennent également.

Dans une forme de réalisation de l'invention, le diéthylèneglycol est utilisé pour l'obtention du polyuréthane soit en tant que diol de bas poids moléculaire, soit comme partie de l'agent d'allongement de chaîne non ionique via l'utilisation de dipropylamine-diéthylèneglycol. Dans le cas où le diéthylèneglycol est utilisé comme diol de bas poids moléculaire, alors, de préférence, le DPA-DEG n'est pas utilisé comme agent d'allongement de chaîne non ionique. De manière analogue, si le DPA-DEG est utilisé comme agent d'allongement de chaîne non ionique, alors, de préférence, on n'utilise pas de diéthylèneglycol comme diol de bas poids moléculaire.

### Procédés de préparation

Les dispersions aqueuses de polyuréthane appropriées pour une utilisation selon l'invention sont susceptibles d'être obtenues par un procédé de préparation comprenant les étapes suivantes :
A) la préparation d'une dispersion aqueuse de polyuréthane par
   1) formation d'un prépolymère à fonctionnalité isocyanate en faisant réagir :
      1 a) un polyesterpolyol, et notamment un polyesterdiol,
      1 b) un polyisocyanate aliphatique ou cycloaliphatique, et
      1c) un diol de bas poids moléculaire, éventuellement substitué par des groupes ioniques ;
   2) un allongement de chaîne du prépolymère par :
      2a) au moins un agent d'allongement de chaîne selon la formule :

         H₂N-R₄-NH₂

         où R₄ représente un radical alkylène ou oxyde d'alkylène non substitué par des groupes ioniques ou potentiellement ioniques, et
      2b) au moins un agent d'allongement de chaîne selon la formule :

         H₂N-R₅-NH₂

         où R₅ représente un radical alkylène substitué par des groupes ioniques ou potentiellement ioniques,
      en présence d'un solvant organique pour former un polyuréthane ;
   3) la dispersion du polyuréthane dans l'eau ; et
   4) l'élimination du solvant organique, ce qui permet d'obtenir une dispersion aqueuse de polyuréthane ; et
le mélange de la dispersion de polyuréthane avec de l'eau ou de l'éthanol.

Plus particulièrement, un procédé de production d'une dispersion de polyuréthane appropriée pour une utilisation dans des produits de maquillage, peut comprendre les étapes suivantes : a) la réaction dans une première étape d'au moins un composé polyesterpolyol et d'un diol de bas poids moléculaire éventuellement substitué par un groupe ionique (des composés dihydroxyle) avec du diisocyanate pour former le prépolymère A), puis b) la dissolution dans une deuxième étape du prépolymère dans un solvant organique et c) la réaction dans une troisième étape de la solution de prépolymère contenant l'isocyanate avec les deux classes d'agents d'allongement de chaîne et, éventuellement, la terminaison de chaîne, d) la formation, dans une quatrième étape, de la dispersion par addition d'eau, et e) l'élimination, dans une cinquième étape, du solvant organique.

Les groupes d'acide sulfonique libres incorporés sont neutralisés entre la troisième et la quatrième étape. Des agents de neutralisation appropriés compris sont les amines primaires, secondaires ou tertiaires. Parmi ceux-ci, on préfère les amines tertiaires substituées par trialkyle. Des exemples de ces amines sont la triméthylamine, la triéthylamine, la triisopropylamine, la tributylamine, la N,N-diméthylcyclohexylamine, la N,N-diméthylstéarylamine, la N,N-diméthylaniline, la N-méthylmorpholine, la N-éthylmorpholine, la N-méthylpipérazine, la N-méthylpyrrolidine, la N-méthylpipéridine, la N,N-diméthyléthanolamine, la N,N-diéthyléthanolamine, la triéthanolamine, la N-méthyldiéthanolamine, le diméthylaminopropanol, la 2-méthoxyéthyldiméthylamine, la N-hydroxyéthylpipérazine, le 2-(2-diméthylaminoéthoxy)-éthanol et la 5-diéthylamino-2-pentanone. Les amines tertiaires préférées sont celles qui ne contiennent pas d'hydrogène(s) actif(s) comme déterminé par le test de Zerewitinoff étant donné que l'hydrogène peut réagir avec les groupes isocyanate des prépolymères ce qui peut provoquer une gélification, la formation de particules insolubles ou la terminaison des chaînes.

Les dispersions de polyuréthane peuvent être produites par ce qu'on appelle le procédé à l'acétone. Dans le procédé à l'acétone, la synthèse des préparations aqueuses de polyuréthane à la base des dispersions selon l'invention est réalisée dans un procédé à étapes multiples.

Dans une première étape, un prépolymère contenant des groupes isocyanate est synthétisé à partir du composé polyesterpolyol, du diisocyanate et du diol de bas poids moléculaire. Les quantités de composants individuels sont calculées de manière telle que la teneur en isocyanate des prépolymères est située entre 1,4 et 5,0% en poids, de préférence entre 2,0 et 4,5% en poids, et de manière particulièrement préférée entre 2,6 et 4,0% en poids. Le diol de bas poids moléculaire est présent en une quantité de 0 à 80% eq. sur base de la quantité d'équivalents NCO, de préférence de 0 à 10% eq.

Le prépolymère obtenu présente la structure : 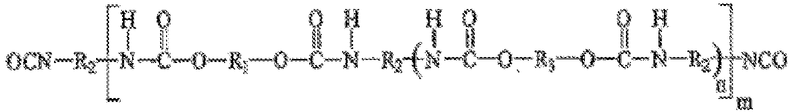 où
R₁ représente un radical radical polyhydroxyle, de préférence dihydroxyle,
R₂ représente un radical hydrocarboné issu d'un polyisocyanate aliphatique ou cycloaliphatique,
R₃ représente un radical hydrocarboné issu d'un diol, éventuellement de faible poids moléculaire, éventuellement substitué par des groupes ioniques,
n vaut 0 à 5, et
m est> 1.

De préférence, n vaut 1 à 3, et m vaut 1 à 5.

Dans une deuxième étape, le prépolymère produit dans l'étape 1 est dissout dans un solvant organique, au moins partiellement miscible à l'eau, ne contenant pas de groupes réactifs avec isocyanate. Le solvant préféré est l'acétone. D'autres solvants tels que par exemple la 2-butanone, le tétrahydrofuranne ou le dioxane ou les mélanges de ces solvants peuvent cependant aussi être utilisés. Les quantités de solvant à utiliser doivent être calculées de manière telle qu'une teneur en solides de 25 à 60% en poids, de préférence de 30 à 50% en poids, de manière particulièrement préférée de 35 à 45% en poids, est obtenue.

Dans une troisième étape, on fait réagir la solution de prépolymère contenant isocyanate avec des mélanges d'agents d'allongement de chaîne à fonctionnalité amino et, éventuellement, des terminaisons de chaîne, pour former le polyuréthane de poids moléculaire élevé. Des quantités suffisantes d'agents d'allongement de chaîne et de terminaison de chaîne sont utilisées de manière telle que le poids moléculaire numérique moyen calculé (Mn) du polyuréthane obtenu est situé entre 10 000 et 100 000 Daltons, de préférence entre 10 000 et 50 000 Daltons. L'agent d'allongement de chaîne non ionique est présent en une quantité de 15 à 90% eq., de préférence de 35,0 à 55% eq., sur base de la quantité résiduelle d'équivalents NCO présents dans le prépolymère. L'agent d'allongement de chaîne ionique est présent en une quantité de 10 à 50% eq., de préférence de 25 à 35% eq., sur base de la quantité résiduelle d'équivalents NCO présents dans le prépolymère. La terminaison de chaîne est présente en une quantité de 0 à 35% eq., de préférence de 20 à 30% eq., sur base de la quantité résiduelle d'équivalents NCO présents dans le prépolymère.

Dans une quatrième étape, le polyuréthane de poids moléculaire élevé est dispersé sous forme d'une dispersion à fines particules par l'addition d'eau à la solution ou de la solution à l'eau.

Dans une cinquième étape, le solvant organique est partiellement ou totalement éliminé par distillation, éventuellement sous pression réduite.

La quantité d'eau dans l'étape quatre est calculée de manière telle que les dispersions aqueuses de polyuréthane selon l'invention affichent une teneur en solides de 23 à 60% en poids, de préférence de 25 à 55% en poids.

Il existe dans le commerce plusieurs grades de ces polymères. Les grades compatibles avec une application cutanée ont fait l'objet d'une première sélection. Parmi les grades compatibles avec une telle application, tous peuvent être utilisés, mais tous ne donnaient pas entière satisfaction dans le cadre de la présente invention. Certains grades se sont avérés sensibilisant, d'autres ne possédaient pas les propriétés requises pour former un film physique présentant une tenue dans le temps convenable, résistant à l'eau.

De manière préférentielle, le polymère dérivé du polyuréthane est un Polyuréthane-32 tel que celui commercialisé sous la dénomination Baycusan C1003 par la société Bayer.

De manière également préférentielle, le polymère dérivé du polyuréthane est constitué d'un prépolymère A) où R2 est un mélange de 1,6- hexaméthylènediisocyanate et d'isophoronediisocyanate, R3 est issu du butane-1,4-diol, l'allongeur de chaîne B) est l'éthylènediamine et l'allongeur de chaîne, C) est le sel de sodium de l'acide N-(2-aminoéthyl)-3-aminoéthanesulfonique

Avec ce grade, le temps de séchage est optimal (inférieur à 5 minutes), le film formé est particulièrement homogène et présente une résistance à l'eau optimale.

En effet, lorsque la composition selon l'invention est formulée avec le Polyuréthane-34 commercialisé par Bayer sous la dénomination commerciale Baycusan C 1000 (notamment constitué d'un prépolymère A) où R2 est le 1,6- hexaméthylènediisocyanate seul), elle forme un film plus cassant, résistant moins bien à l'eau que lorsqu'elle est formulée avec le Polyuréthane-32 (Baycusan C1003).

Lorsque la composition selon l'invention est formulée avec le Polyuréthane-34 commercialisé par Bayer sous la dénomination commerciale Baycusan C 1001 (notamment constitué d'un prépolymère A) où R2 est le 1,6- hexaméthylènediisocyanate seul), elle présente un temps de séchage moins optimal pour ce type d'application (supérieur à 5 minutes). De plus, l'aspect du film formé après séchage n'est pas aussi homogène que celui obtenu avec le Polyuréthane-32 (Baycusan C1003).

Le Polyuréthane-35 commercialisé par Bayer sous la dénomination commerciale Baycusan C 1004 (notamment constitué d'un prépolymère A) où R2 est le dicyclohexylméthane diisocyanate seul) forme quant à lui un film qui résiste moins bien à l'eau que le film obtenu avec le Polyuréthane-32 (Baycusan C1003).

De préférence, le polymère dérivé du polyuréthane est présent dans la composition selon l'invention en une teneur en matières sèches allant de 23 % à 60 % en poids, de préférence allant de 25 % à 55 % en poids, par rapport au poids total de la composition.

### Acide hydroxamique

La composition selon l'invention comprend au moins un acide hydroxamique, ses sels et/ou ses complexes. Ces composés sont notamment décrits en détails dans le document WO 2009/070736.

Dans le cadre de la présente demande, l'acide hydroxamique est de préférence un acide alkyl hydroxamique.

L'acide hydroxamique, notamment l'acide alkyl hydroxamique, peut être utilisé dans les compositions de l'invention sous une forme libre (non neutralisée) ou sous forme de sel (neutralisée).

Les termes «acide hydroxamique» et «acide alkyl hydroxamique» incluent donc, au sens de la présente invention, les formes libres, les sels ou complexes desdits acides, ainsi que leurs précurseurs, et les sels et complexes de ces précurseurs.

Les acides alkyl hydroxamiques sont des composés de formule (I): 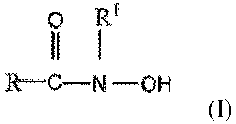 dans laquelle R est une chaîne carbonée linéaire ou ramifiée, substituée ou non, comprenant de deux à vingt-deux atomes de carbone, qui peut être interrompue par un ou plusieurs atomes d'oxygène, et peut inclure des liaisons carbones saturées ou insaturées.

Les groupes R peuvent inclure, par exemple, les groupes alkyle, alcényl, alcynyle, alcoxy, alcénoxy, alcynoxy et des groupes similaires, linéaires ou ramifiés, pouvant être fonctionnalisés avec des groupes de substitution incluant les groupements hydroxy ou d'autres groupements fonctionnels pharmaceutiquement ou cosmétiquement acceptables.

R1 peut être l'hydrogène ou le radical R précédemment décrit.

L'acide alkyl hydroxamique peut donc notamment comprendre une chaîne carbonée linéaire ou ramifiée, saturée ou insaturée, substituée ou non, comprenant de deux à vingt-deux atomes de carbone, et de préférence de 6 à 12 atomes de carbone.

Les chaînes carbonées peuvent comprendre des insaturations éthyléniques, et/ou être porteuses de groupements chimiques particuliers, choisis selon les propriétés finales souhaitées pour l'acide alkyl hydroxamique.

Par exemple, la présence de groupements hydroxy sur la chaîne peut permettre une meilleure compatibilité du composé avec l'eau.

Les chaînes carbonées peuvent également comprendre d'autres groupes fonctionnels pharmaceutiquement ou cosmétiquement acceptables.

Les acides alkyl hydroxamiques peuvent être synthétisés à partir d'huiles naturelles utilisant la catalyse lipase ainsi que d'autres techniques de synthèse hydroxamique connues de l'homme de l'art.

De préférence, l'acide alkyl hydroxamique répond à la formule (I) dans laquelle R est une chaîne carbonée linéaire ou ramifiée, saturée ou insaturée, substituée ou non, comprenant de deux à vingt-deux atomes de carbone, et de préférence de 6 à 12 atomes de carbone, et R1 est l'hydrogène.

L'acide alkyl hydroxamique est, de préférence, choisi dans le groupe constitué, de manière non limitative, par l'acide hexanohydroxamique, l'acide caprylohydroxamique, l'acide caprohydroxamique, l'acide laurohydroxamique et leurs mélanges.

Selon un mode particulièrement préféré de l'invention, l'acide alkyl hydroxamique est choisi parmi l'acide caprylohydroxamique (i.e. présentant une chaîne hydrocarbonée linéaire comprenant huit atomes de carbone) et l'acide caprohydroxamic (i.e. présentant une chaîne hydrocarbonée linéaire comprenant dix atomes de carbone).

Selon un mode plus préféré de réalisation de l'invention, l'acide alkyl hydroxamique est l'acide caprylohydroxamique.

Il convient de noter que les précurseurs de l'acide alkyl hydroxamique, tels que les acides hydroxy en combinaison avec, par exemple, le chlorhydrate d'hydroxylamine ou des composés similaires, qui peuvent réagir au sein de la composition de l'invention pour former l'acide alkyl hydroxamique, ou des sels et/ou complexes de celui-ci peuvent être introduits dans la composition selon l'invention en lieu et place de l'acide alkyl hydroxamique.

Dans le cadre de la présente invention, l'acide hydroxamique est présent en une teneur allant de 0,01 à 10% en poids, de préférence de 0,1 à 8 %, et plus préférentiellement de 0,1 à 5% en poids, par rapport au poids total de la composition.

En effet, il a été montré dans le cadre de la présente demande que, de manière tout à fait surprenante, l'ajout d'acide hydroxamique à des compositions comprenant un polymère dérivé du polyuréthane et un épaississant permettait de stabiliser dans le temps la viscosité des compositions.

Selon un mode particulier de réalisation, l'acide hydroxamique est introduit dans la composition selon l'invention sous la forme d'un pré-mélange avec au moins un alcool.

Les alcools particulièrement préférés pour former un pré-mélange avec l'acide hydroxamique sont des diols vicinaux.

On parle de diol vicinal lorsque les deux groupes hydroxyles sont en position vicinale, c'est-à-dire attachés à des atomes de carbone adjacents.

A titre d'exemple de diol vicinal utilisable dans le cadre de la présente demande, on peut citer de manière non limitative l'éthylène glycol (l'éthane-1,2-diol), le propylène glycol (propane-1,2-diol), le 1,2-pentanediol, le 1,2-hexanediol, le caprylyl glycol, le 1,2-décanediol ainsi que les dérivés de la glycérine tels que les monoéthers de glycéryle, par exemple l'éthylhexylglycerine, le monolaurate de glycéryle, le mono caproate, ou monocaprylate glycéryle.

Les compositions selon l'invention comprennent de préférence des diols vicinaux en une teneur allant de 0,1 à 45% en poids, de préférence de 0,5 à 20% en poids, par rapport au poids total de la composition.

Selon un mode préféré de réalisation, l'acide hydroxamique est introduit dans la composition selon l'invention sous la forme d'un pré-mélange avec du caprylyl glycol et de la glycérine.

Des pré-mélanges d'acide hydroxamique avec du caprylyl glycol et de la glycérine pouvant convenir dans le cadre de la présente demande sont notamment les produits commercialisés par la société Inolex sous la dénomination Spectrastat.

En particulier, il a été montré dans le cadre de la présente demande que, de manière tout à fait surprenante, l'ajout d'acide hydroxamique sous forme pré-mélange avec du caprylyl glycol et de la glycérine tel que celui commercialisé par la société Inolex sous la dénomination Spectrastat à des compositions comprenant un polymère dérivé du polyuréthane et un épaississant permettait de stabiliser dans le temps la viscosité des compositions.

### Épaississant

La composition selon l'invention comprend au moins un agent épaississant. Cet agent épaississant peut être choisi parmi les agents épaississants minéraux ou organiques, polymériques ou non polymériques, et leurs mélanges.

On entend par agent épaississant, un composé qui modifie la rhéologie du milieu dans lequel il est incorporé.

Ainsi l'agent épaississant peut être choisi parmi les gommes végétales, les acides polyacryliques, le polyéthylène glycol.

Parmi les gommes végétales, on peut citer, à titre d'exemple, l'agar agar, l'acide alginique, le carraghénane, la gomme arabique, la gomme tragacanthe, la gomme karaya, la gomme guar, les béta-glucanes, la gomme de caroube, le chiclé, les glucomannanes, la gomme xanthanne.

Les compositions selon la présente demande peuvent aussi contenir au moins un épaississant hydrophile choisi parmi :
- les homo- ou copolymères d'acides acrylique ou méthacrylique ou leurs sels et leurs esters et en particulier les produits vendus sous les dénominations VERSICOL F ou VERSICOL K par la société ALLIED COLLOID, UTRAHOLD 8 par la société CIBA-GEIGY, les acides polyacryliques de type SYNTHALEN K,
- les copolymères d'acide acrylique et d'acrylamide vendus sous la forme de leur sel de sodium sous les dénominations RETEN par la société HERCULES, les sels de sodium d'acides polyhydroxycarboxyliques vendus sous la dénomination HYDAGEN F par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL ou SIMULGEL commercialisés par la société SEPPIC, ou
- les copolymères AMPS/méthacrylates d'alkyle polyoxyéthylénés (réticulés ou non), et leurs mélanges.

De préférence, l'épaississant utilisé dans le cadre de la présente invention est un copolymère acide polyacryliques/acrylates d'alkyle, tel que le PEMULEN TR 1NF.

Selon un mode particulier de réalisation, l'épaississant est différent du p-isononylphenoxypoly(glycidol) par exemple commercialisé sous la dénomination OLIN 10G par la société OLIN.

L'épaississant représente 0,01 à 8%, de préférence 0,03 à 5%, plus préférentiellement 0,06 à 2% en poids par rapport au poids total de la composition.

### Milieu pharmaceutiquement acceptable

La composition selon l'invention comprend un milieu pharmaceutiquement acceptable comprenant au moins un solvant.

Par milieu pharmaceutiquement acceptable, on entend, au sens de la présente demande, un milieu compatible avec la peau.

Tout solvant compatible avec le polymère de polyuréthane peut être utilisé, de préférence on utilisera l'eau.

Le solvant peut représenter de 10 à 75% en poids, de préférence de 25 à 65% en poids, et plus préférentiellement de 30 à 55% en poids, par rapport au poids total de la composition.

Le polymère de polyuréthane est généralement commercialisé en phase aqueuse (en particulier pour ce qui concerne les applications dermato-cosmétiques).

Dans le cas de la présente invention, le grade de polymère préféré, à savoir le Polyuréthane-32 a un extrait sec de 50 %, ce qui correspond à une teneur en eau de 50%.

### Polymère filmogène secondaire

La composition selon l'invention peut comprendre, en plus du polymère dérivé du polyuréthane, un polymère filmogène secondaire.

Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide, le copolymère de PolyVinylMéthyl éther et de Maleic Anhydride (PVM/MA).

Comme polymère filmogène, on peut notamment utiliser la nitrocellulose RS 1/8 sec ; RS [1/4] sec. ; [1/2] sec. ; RS 5 sec. ; RS 15 sec. ; RS 35 sec. ; RS 75 sec. ; RS 150 sec ; AS [1/4] sec. ; AS [1/2] sec. ; SS [1/4] sec. ; SS [1/2] sec. ; SS 5 sec., notamment commercialisée par la société HERCULES, ou la nitrocellulose DHL 120/170 IPA commercialisée par la société NOBEL ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Le polymère filmogène secondaire peut être présent dans la composition selon l'invention en une teneur en matières sèches allant de 5 à 25% en poids, de préférence allant de 5 à 10% en poids, par rapport au poids total de la composition.

### Agent auxiliaire de filmification

Pour améliorer les propriétés filmogènes de la composition, un agent auxiliaire de filmification peut avantageusement être ajouté.

L'agent auxiliaire de filmification est, bien évidemment, différent du solvant organique présent dans le milieu pharmaceutiquement acceptable.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :
- les alcools gras comme l'octyldodécanol, le 2-butyloctanol, le 2-hexyl décanol, le 2-undécylpentadécanol, l'alcool oléique ;
- les glycols et leurs dérivés, tels que la glycérine, le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les acides gras tels que l'acide oléique, l'acide linoléique, l'acide linolénique ;
- les esters de glycol tels que la triacétine (ou triacétate de glycéryle) ;
- les dérivés de propylène glycol et en particulier le propylène glycol phényléther, le propylène glycol diacétate, le dipropylène glycol éthyléther, le tripropylène glycol méthyléther, le propylène glycol butyléther ;
- les esters d'acides, notamment carboxyliques, tels que les citrates, les phtalates, les adipates, les carbonates, les tartrates, les phosphates, les sébacates et en particulier les esters d'acide monocarboxylique tels que l'isononanoate d'isononyle, l'érucate d'oléyle ou le néopentanoate d'octyl-2-docécyle ;
- les dérivés oxyéthylénés, tels que les huiles oxyéthylénées, notamment les huiles végétales, telles que l'huile de sésame, l'huile de ricin, l'huile d'amande, l'huile de canola, l'huile de noisette, l'huile de pistache, l'huile de lin, l'huile de bourrache, l'huile de chanvre, l'huile de jojoba, l'huile de tournesol, l'huile de germe de blé, l'huile de maïs et/ou de germe de maïs, l'huile d'arachide, l'huile d'avocat, l'huile de carthame, l'huile de colza, l'huile d'olive, l'huile d'argan, l'huile de tournesol, l'huile de pépin de raisin, l'huile de soja, l'huile de noix, l'huile de pépin de de courge, l'huile de palme, l'huile de coprah, et leurs mélanges. L'huile peut également être un dérivé d'une des huiles végétales citées précédemment. Il peut s'agir d'huile hydrogénée ou non, peroxydée ou non.
et leurs mélanges.

Selon un mode préféré de réalisation, l'agent auxiliaire de filmification est choisi parmi les dérivés oxyéthylénés, tels que les huiles oxyéthylénées, notamment les huiles végétales, telles que l'huile de ricin.

Par exemple, la teneur en agent auxiliaire de filmification peut aller de 0,05 à 5% et en particulier de 0.1 à 3% en poids par rapport au poids total de la composition.

### Additifs

La composition selon l'invention peut comprendre un ou plusieurs additifs pharmaceutiquement acceptables, comme par exemple les parfums, les arômes, les colorants, les pigments, les agents matifiants, les agents rhéologiques, les conservateurs, les vitamines, les huiles essentielles et les agents actifs, notamment choisis parmi les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques et les filtres solaires.

En particulier, les actifs pouvant être introduits dans la composition selon l'invention peuvent être choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que l'Aciclovir, le Famciclovir, le Ritonavir ;
- les antifongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, la metformine, les extraits de Centella Asiatica, la papaïne, les silicones, les huiles essentielles de thym, de niaouli, de romarin et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, l'Allantoïne ;
- les agents hydratants tels que l'acide hyaluronique, l'urée, le glycérol, les acides gras, modulateurs des aquaporines, les huiles végétales, le chitosan, certains sucres dont le sorbitol, les beurres et les cires ;
- les agents dépigmentants tels que l'acide kojique (Kojic Acid SL® - Quimasso (Sino Lion)), l'Arbutine (Olevatin® - Quimasso (Sino Lion)), le mélange de palmitoylpropyl de sodium et d'extrait de nénuphar blanc (Sepicalm® - Seppic), l'undécylénoyl phénylalanine (Sepiwhite® - Seppic), l'extrait de réglisse obtenue par fermentation d'Aspergillus et éthoxydiglycol (Gatuline Whitening® - Gattefossé), l'acide octadécènedioïque (ODA White® - Sederma), l'alpha-arbutin (Alpha-arbutin®, SACI-CFPA (Pentapharm)), l'extrait aqueux de feuilles Arctophylos Uva Ursi (Melfade-J® - SACI-CFPA (Pentapharm)), le mélange de plante complexe Gigawhite® (SACI-CFPA (Alpaflor)), la diacétyl boldine (Lumiskin® - Sederma), l'extrait de mandarine du Japon (Melaslow® - Sederma), le mélange d'extrait de citron enrichi en acide citrique et d'extrait de concombre (Uninontan®U-34 - Unipex), le mélange d'extrait de Rumex occidentalis et de vitamine C (Tyrostat® 11 - Unipex), des oligopeptides (Mélanostatine 5® - Unipex), le dipalmitate kojique (KAD-15® - Quimasso (Sino Lion)), le complexe d'origine naturelle Vegewhite® de LCW, des extraits de germe de blé (Clariskin® II - Silab), l'éthyldiamine triacétate (EDTA);
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline® (Sederma), les protéases obtenues par fermentation de Bacillus Subtilis, le produit Linked-Papain® (SACI-CFPA), la papaïne (enzyme protéolytique issue du fruit de papaye);
- les actifs restructurants (par exemple resctructurants des phanères) tels que les dérivés de silice, la vitamine E, la camomille, le calcium, l'extrait de prêle, le Lipester de soie;
- les anesthésiques tels que la benzocaïne, la lidocaïne, la dibucaïne, le chlorhydrate de pramoxine, la bupivacaïne, la mepivacaïne, la prilocaïne, l'étidocaïne ;
- les filtres solaires, tels que les filtres chimiques (Oxybenzone, Sulisobenzone, Dioxybenzone, Tinosorb S®, Avobenzone, p-méthoxycinnamate de 2-éthoxyéthyle, Uvinul® A+, Mexoryl® XL, Méthoxycinnamate ou octinoxate d'octyle, Salicylate ou octisalate d'octyle, octyl triazone ou Uvinul® T 150, salicylate de méthyle, meradimate, enzacamène, MBBT ou Tinosorb® M, cyanophénylcinnamate d'octyle ou Parsol® 340, Acide para-aminobenzoïque, Ensulizole, Parsol® SLX ou Polysiloxane-15 ou Benzylidène malonate polysiloxane, salicylate de triéthanolamine ou salicylate de trolamine, Mexoryl® SX ou acide téréphtalylidène dicamphosulfonique) et les filtres minéraux (oxydes de Zinc, dioxyde de titane, kaolin, ichtyol).

### Préparation d'un film

Selon un mode particulier de réalisation, l'invention a pour objet un procédé d'obtention d'un film à partir d'une composition filmogène telle que décrite précédemment, caractérisé en que :
i. on applique une composition telle que décrite précédemment sur les tissus tels que la peau, les phanères ou les muqueuses de manière à former un film liquide uniforme,
ii. on laisse sécher pendant 1 à 5 minutes.

L'épaisseur du film liquide uniforme est par exemple supérieure à 500 nm, de préférence comprise entre 1 et 300 µm, plus préférentiellement comprise entre 2 et 200 µm.

L'invention a aussi pour objet un film susceptible d'être obtenu à partir du procédé décrit ci-dessus.

### Utilisation de l'acide hydroxamique

Selon un mode particulier de réalisation, l'invention a pour objet l'utilisation de l'acide hydroxamique pour stabiliser la viscosité d'une composition comprenant au moins un polymère filmogène dérivé du polyuréthane et un épaississant.

En particulier, l'invention a pour objet l'utilisation de l'acide hydroxamique pour stabiliser la viscosité de la composition comprenant
- au moins un polymère filmogène dérivé du polyuréthane présent dans la composition en une teneur en matières sèches allant de 23 % à 60 % en poids, de préférence allant de 25 % à 55 % en poids, par rapport au poids total de la composition, et
- au moins un épaississant.

La présente invention est illustrée plus en détails dans les exemples non limitatifs décrits ci-après.

### Exemples 1 à 4

On a préparé les 4 compositions suivantes :

| | | **Example 1 comparatif** | **Exempe 2 selon l'invention** | **Example 3 comparatif** | **Example 4 Selon l'invention** |
|---|---|---|---|---|---|
| **Nom commercial** | **Nom INCI** | **% massique** | **% massique** | **% massique** | **% massique** |
| Dispersion aqueuse de polymère dérivé du polyuréthane Baycusan C 1003⁽¹⁾ commercialisé par la société Bayer | | 97.8 | 97.55 | 98.97 | 97.77 |
| Spectrastat commercialisé par la société Inolex | Caprylhydroxamic Acid / Caprylyl Glycol / Glycerin | **1.2** | **1.2** | **-** | **1.2** |
| Huile de germe de blé commercialisé par la société Prod'hyg laboratoires | | 1 | 1 | 1 | 1 |
| Pemulen TR-1NF commercialisé par la société Lubrizol | Acrylates/C10-30 Alkyl Acrylate Crosspolymer | - | - | **0.03** | **0.03** |
| Gomme xanthane commercialisée par la société Jungbunzlauer | | - | **0.25** | - | - |

| | | | | | |
|---|---|---|---|---|---|
| ⁽¹⁾ comprenant 50% +/- 2% de matières solides en dispersion dans l'eau. | | | | | |

Les compositions des exemples 1 à 4 ont été préparées en mélangeant le polymère dérivé du polyuréthane (commercialisée en solution) et l'huile de germe de blé sous agitation (700 rpm) pendant 5 minutes. Une fois que le mélange est homogène, le Spectrastat est ajouté sous vive agitation (900 rpm) et on laisse homogénéiser 10 minutes, puis, toujours sous vive agitation, on ajoute le Pemulen TR-1 NF lorsqu'il est présent. On laisse homogénéiser 10 minutes à 1100 rpm puis 20 minutes à 1400 rpm.

L'ensemble de ces étapes se sont déroulées sous agitateur à hélice jusqu'à dissolution complète des constituants.

On a étudié l'évolution de la viscosité à 40°C des exemples 1 à 4.

La viscosité des compositions selon les exemples 1 à 4 est mesurée selon le protocole suivant à t0, t=1 mois, t=2 mois, t=3 mois, t=6 mois. Les compositions sont placées dans des étuves à 40°C:
- Viscosimètre Brookfield LVDV ;
- mesure de la viscosité à 6rpm pendant 1 minute avec les mobiles LV2 à LV4 suivant la viscosité du mélange analysé.

| Exemples | Viscosité (cPs) à t0 | Viscosité (cPs) à t1 mois | Viscosité (cPs) à t2 mois | Viscosité (cPs) à t3 mois | Viscosité (cPs) à t6 mois |
|---|---|---|---|---|---|
| Exemple 4 selon l'invention | 12737 | 11038 | 9478 | 7825 | 5952 |
| Exemple 1 comparatif | 1032 | - | - | - | - |
| Exemple 2 selon l'invention | 15707 | 14784 | 13864 | 13224 | 12144 |
| Exemple 3 comparatif | 14637 | 4239 | 2233 | 1387 | 1087 |

Une viscosité inférieure à 5000cPs n'est pas acceptable car le film formé est trop fin (déceptif en terme de tenue dans le temps) et ne couvre pas efficacement les affections cutanés. Le film formé n'est pas suffisamment résistant. Ainsi, l'exemple 1 comparatif sans épaississant est jugé déceptif dès le t0 et n'a donc pas été placé en stabilité.

Comme on peut le constater, la présence d'acide hydroxamique contenu dans le Spectrastat dans les compositions 2 et 4 selon l'invention permet, de manière surprenante et inattendue, de stabiliser dans le temps la viscosité des compositions comprenant un polymère filmogène dérivé du polyuréthane en association avec un épaississant, par rapport à la composition comparative de l'exemple 3.

### Exemples 5 à 7

| | | **Exemple 5 comparatif** | **Exemple 6 selon l'invention** | **Exemple 7 Selon l'invention** | **Exemple 8 comparatif** |
|---|---|---|---|---|---|
| **Nom commercial** | **Nom INCI** | **% massique** | **% massique** | **% massique** | **% massique** |
| Dispersion aqueuse de polymère dérivé du polyuréthane Baycusan C 1003⁽¹⁾ commercialisé par la société Bayer | | 39,108 | 48,885 | 97,523 (Baycusan concentré avec un poids sec à 56%)⁽²⁾ | 97,77 (Baycusan concentré avec un poids sec à 65,8%)⁽²⁾ |
| Spectrastat commercialisé par la société Inolex | Caprylhydroxamic Acid / Caprylyl Glycol / Glycerin | 0,480 | 0,600 | 1,333 | 1,20 |
| Huile de germe de blé commercialisé par la société Prod'hyg laboratoires | | 0,400 | 0,500 | 1,111 | 1,00 |
| Pemulen TR-1NF commercialisé par la société Lubrizol | Acrylates/C10-30 Alkyl Acrylate Crosspolymer | 0,012 | 0,015 | 0,033 | 0,033 |
| Eau purifiée | NA | 60,000 | 50,000 | - | - |

| | | | | | |
|---|---|---|---|---|---|
| ⁽¹⁾ comprenant 50% +/- 2% de matières solides en dispersion dans l'eau, ⁽²⁾ le Baycusan C 1003 comprend 50% en poids sec. Ainsi pour obtenir une quantité supérieure à 50%, on a évaporé l'eau jusqu'à obtenir la concentration en polyuréthane souhaitée. | | | | | |

On a évalué la qualité des films obtenus avec les compositions des exemples 5 à 7.

Les films ont été obtenus après enduction sur une épaisseur d'1 mm des produits fabriqués sur un film de polyester siliconé, à l'aide d'une table d'enduction.

L'exemple 5 comparatif comprenant une teneur en matière sèche inférieure à 20% en poids de polymère dérivé du polyuréthane forme un film fin, cassant et non-homogène.

L'exemple 6 selon l'invention comprenant une teneur en matière sèche d'environ 25% de polymère dérivé du polyuréthane forme un film homogène et résistant à l'eau.

L'exemple 7 selon l'invention comprenant une teneur en matière sèche d'environ 55% de polymère dérivé du polyuréthane forme un film homogène et résistant à l'eau.

L'exemple 8 comparatif comprenant une teneur en matière sèche supérieure à 60% en poids de polymère dérivé du polyuréthane ne permet pas d'obtenir un film utilisable selon l'invention. La composition obtenue est trop épaisse et ne peut pas être appliquée correctement.

### Exemples 9 à 12

On a préparé les 4 compositions suivantes :

| | | **Exemple 9** | **Exemple 10** | **Example 11** | **Example 12** |
|---|---|---|---|---|---|
| **Nom commercial** | **Nom INCI** | **% massique** | **% massique** | **% massique** | **% massique** |
| Dispersion aqueuse de polymère dérivé du polyuréthane Baycusan C1000⁽¹⁾ commercialisé par la société Bayer | Polyurethane-34 | 97.77 | | | |
| Dispersion aqueuse de polymère dérivé du polyuréthane Baycusan C1001⁽²⁾ commercialisé par la société Bayer | Polyurethane-34 | | 97.77 | | |
| Dispersion aqueuse de polymère dérivé du polyuréthane Baycusan C1003⁽³⁾ commercialisé par la société Bayer | Polyurethane-32 | | | | 97.77 |
| Dispersion aqueuse de polymère dérivé du polyuréthane Baycusan C1004⁽⁴⁾ commercialisé par la société Bayer | Polyurethane-35 | | | 97.77 | |
| Spectrastat commercialisé par la société Inolex | Caprylhydroxamic Acid / Caprylyl Glycol / Glycerin | 1.2 | 1.2 | 1.2 | 1.2 |
| Huile de germe de blé commercialisé par la société Prod'hyg laboratoires | | 1 | 1 | 1 | 1 |
| Pemulen TR-1NF commercialisé par la société Lubrizol | Acrylates/C10-30 Alkyl Acrylate Crosspolymer | 0.03 | 0.03 | 0.03 | 0.03 |

| | | | | | |
|---|---|---|---|---|---|
| ⁽¹⁾ comprenant 40% +/- 2% de matières solides en dispersion dans l'eau, ⁽²⁾ comprenant 32% +/- 2% de matières solides en dispersion dans l'eau, ⁽³⁾ comprenant 50% +/- 2% de matières solides en dispersion dans l'eau, ⁽⁴⁾ comprenant 41% +/- 2% de matières solides en dispersion dans l'eau. | | | | | |

Les compositions des exemples 9 à 12 ont été préparées en mélangeant le polymère dérivé du polyuréthane (commercialisée en solution) et l'huile de germe de blé sous agitation (700 rpm) pendant 5 minutes. Une fois que le mélange est homogène, le Spectrastat est ajouté sous vive agitation (900 rpm) et on laisse homogénéiser 10 minutes, puis, toujours sous vive agitation, on ajoute le Pemulen TR-1 NF. On laisse homogénéiser 10 minutes à 1100 rpm puis 20 minutes à 1400 rpm.

L'ensemble de ces étapes se sont déroulées sous agitateur à hélice jusqu'à dissolution complète des constituants.

La composition filmogène de l'exemple 12 constitue le mode préféré de réalisation de l'invention. Elle présente un temps de séchage optimal lorsqu'elle est appliquée sur la peau (inférieur à 5minutes), et forme un film particulièrement homogène et présentant une excellente résistance à l'eau.

La composition filmogène selon l'exemple 9 forme un film un peu plus cassant que celui obtenu avec la composition selon l'exemple 12 et résistant un peu moins bien à l'eau comparativement.

La composition filmogène selon l'exemple 10 présente un temps de séchage supérieur à celui de l'exemple 12 (supérieur à 5 minutes). Le film obtenu après séchage est un peu moins homogène que celui obtenu avec la composition de l'exemple 12.

La composition filmogène selon l'exemple 11 forme un film résistant un peu moins bien à l'eau que celui obtenu avec la composition de l'exemple 12.

## Revendications

1. Composition liquide filmogène **caractérisée en ce qu'**elle comprend :
- au moins un polymère filmogène dérivé du polyuréthane présent dans la composition en une teneur en matières sèches allant de 23 % à 60 % en poids, de préférence allant de 25 % à 55 % en poids, par rapport au poids total de la composition,
- au moins un acide hydroxamique, ses sels et/ou ses complexes et
- au moins un épaississant.

2. Composition filmogène selon la revendication 1, **caractérisée en ce que** le polymère filmogène dérivé du polyuréthane répond au nom INCI Polyuréthane-32

3. Composition filmogène selon la revendication 1, **caractérisée en ce que** le polymère filmogène dérivé du polyuréthane est constitué d'un prépolymère
A) de formule : 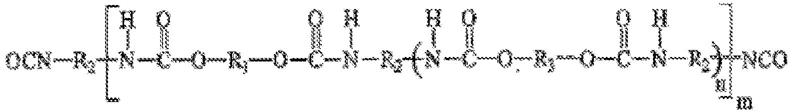 où
R₁ représente un radical radical polyhydroxyle, de préférence dihydroxyle,
R₂ représente un radical hydrocarboné issu d'un polyisocyanate aliphatique ou cycloaliphatique,
R₃ représente un radical hydrocarboné issu d'un diol, éventuellement de faible poids moléculaire, éventuellement substitué par des groupes ioniques,
n vaut 0 à 5, et
m est > 1 ;
B) d'au moins un agent d'allongement de chaîne selon la formule :
H₂N-R₄-NH₂
où R₄ représente un radical alkylène ou oxyde d'alkylène non substitué par des groupes ioniques ou potentiellement ioniques ; et
C) d'au moins un agent d'allongement de chaîne de formule :
R₅-NH-R₅'-NH₂
où R₅ représente un radical alkylène substitué par des groupes ioniques ou potentiellement ioniques, et R₅' représente un radical alkyle, notamment en C₂-C₄.

4. Composition filmogène selon la revendication précédente, **caractérisée en ce que** R2 est un mélange de 1,6- hexaméthylènediisocyanate et d'isophoronediisocyanate, R3 est issu du butane-1,4-diol, l'allongeur de chaîne B) est l'éthylènediamine et l'allongeur de chaîne C) est le sel sodique de l'acide N-(2-aminoéthyl)-3-aminoéthanesulfonique.

5. Composition filmogène selon l'une des revendications précédentes, **caractérisée en ce que** l'acide hydroxamique est présent en une teneur allant de 0,01 à 10% en poids, de préférence de 0,1 à 8 %, et plus préférentiellement de 0,1 à 5% en poids, par rapport au poids total de la composition.

6. Composition filmogène selon l'une des revendications précédentes, **caractérisée en ce que** l'acide hydroxamique est choisi dans le groupe constitué par l'acide hexanohydroxamique, l'acide caprylohydroxamique, l'acide caprohydroxamique, l'acide laurohydroxamique et leurs mélanges, de préférence l'acide hydroxamique est l'acide caprylohydroxamique.

7. Composition filmogène selon l'une des revendications précédentes, **caractérisée en ce que** l'épaississant représente 0,01 à 8%, de préférence 0,01 à 5%, plus préférentiellement 0,01 à 2% en poids par rapport au poids total de la composition.

8. Procédé d'obtention d'un film à partir d'une composition filmogène selon l'une quelconque des revendications 1 à 7, caractérisé en que :
i. on applique une composition selon l'une quelconque des revendications 1 à 6 sur les tissus tels que la peau, les phanères ou les muqueuses de manière à former un film liquide uniforme,
ii. on laisse sécher pendant 1 à 5 minutes.

9. Procédé selon la revendication 8, **caractérisé en ce que** l'épaisseur du film liquide uniforme est supérieure à 500 nm, de préférence comprise entre 1 et 300 µm, plus préférentiellement comprise entre 2 et 200 µm.

10. Film susceptible d'être obtenu à partir du procédé selon l'une des revendications 8 ou 9.

11. Utilisation de l'acide hydroxamique pour stabiliser la viscosité d'une composition comprenant au moins un polymère filmogène dérivé du polyuréthane et un épaississant, de préférence une composition selon l'une des revendications 1 à 7.

## Patentansprüche

1. Flüssige filmbildende Zusammensetzung, **dadurch gekennzeichnet, dass** sie umfasst:
- mindestens ein filmbildendes Polymer, das abgeleitet ist von dem in der Zusammensetzung enthaltenen Polyurethan in einem Trockenmassegehalt von 23 bis 60 Gew.-%, vorzugsweise 25 bis 55 Gew.-%, bezogen auf das Gesamtgewicht der Zusammensetzung;
- mindestens eine Hydroxamsäure, ihre Salze und/oder Komplexe; und
- mindestens ein Verdickungsmittel.

2. Filmbildende Zusammensetzung nach Anspruch 1, **dadurch gekennzeichnet, dass** das von Polyurethan abgeleitete filmbildende Polymer der Bezeichnung INCI Polyurethan-32 entspricht.

3. Filmbildende Zusammensetzung nach Anspruch 1, **dadurch gekennzeichnet, dass** das von Polyurethan abgeleitete filmbildende Polymer gebildet ist aus einem Präpolymer
A) der Formel 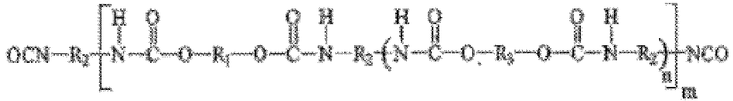 worin
R₁ einen Polyhydroxylrest, vorzugsweise Dihydroxyl, darstellt,
R₂ einen Kohlenwasserstoffrest darstellt, der sich von einem aliphatischen oder cycloaliphatischen Polyisocyanat ableitet,
R₃ einen Kohlenwasserstoffrest darstellt, der sich von einem Diol ableitet, gegebenenfalls mit niedrigem Molekulargewicht, gegebenenfalls mit ionischen Gruppen substituiert,
n 0 bis 5 ist und
m > 1 ist;
B) mindestens einem Kettenverlängerer der Formel:
H₂N-R₄-NH₂,
worin R₄ einen Alkylen- oder Alkylenoxidrest darstellt, der nicht mit ionischen oder potenziell ionische Gruppen substituiert ist; und
C) mindestens einem Kettenverlängerer der Formel:
R₅-NH-R₅'-NH₂
worin R₅ einen Alkylenrest darstellt, der mit ionischen oder potentiell ionischen Gruppen substituiert ist, und R₅' einen Alkylrest darstellt, insbesondere einen C₂-C₄-Alkylrest.

4. Filmbildende Zusammensetzung nach dem vorstehenden Anspruch, **dadurch gekennzeichnet, dass** R₂ eine Mischung aus 1,6-Hexamethylendiisocyanat und Isophorondiisocyanat ist, R₃ von Butan-1,4-diol abgeleitet ist, der Kettenverlängerer B) Ethylendiamin ist und der Kettenverlängerer C) das Natriumsalz von N-(2-Aminoethyl)-3-aminoethansulfonsäure ist.

5. Filmbildende Zusammensetzung nach einem der vorstehenden Ansprüche,
**dadurch gekennzeichnet, dass** die Hydroxamsäure in einer Menge im Bereich von 0,01 bis 10 Gew.-%, bevorzugt von 0,1 bis 8 Gew.-%, und weiter bevorzugt von 0,1 bis 5 Gew.-%, bezogen auf das Gesamtgewicht der Zusammensetzung, enthalten ist.

6. Filmbildende Zusammensetzung nach einem der vorstehenden Ansprüche,
**dadurch gekennzeichnet, dass** die Hydroxamsäure ausgewählt ist aus der Gruppe bestehend aus Hexanohydroxamsäure, Caprylohydroxamsäure, Caprohydroxamsäure, Laurohydroxamsäure und Mischungen derselben, wobei die Hydroxamsäure vorzugsweise Caprylohydroxamsäure ist.

7. Filmbildende Zusammensetzung nach einem der vorstehenden Ansprüche,
**dadurch gekennzeichnet, dass** das Verdickungsmittel 0,01 bis 8 Gew.-%, bevorzugt 0,01 bis 5 Gew.-%, weiter bevorzugt 0,01 bis 2 Gew.-%, bezogen auf das Gesamtgewicht der Zusammensetzung, darstellt.

8. Verfahren zur Herstellung eines Films aus einer filmbildenden Zusammensetzung nach einem der Ansprüche 1 bis 7, **dadurch gekennzeichnet, dass**:
i. eine Zusammensetzung nach einem der Ansprüche 1 bis 6 auf Gewebe wie Haut, Hautanhangsgebilde oder Schleimhäute aufgebracht wird, um einen gleichmäßigen Flüssigkeitsfilm zu bilden,
ii. für 1 bis 5 Minuten trocknen gelassen wird.

9. Verfahren nach Anspruch 8, **dadurch gekennzeichnet, dass** die Dicke des gleichmäßigen Flüssigkeitsfilms größer als 500 nm ist, bevorzugt zwischen 1 und 300 µm, weiter bevorzugt zwischen 2 und 200 µm.

10. Film, erhältlich aus dem Verfahren nach einem der Ansprüche 8 oder 9.

11. Verwendung von Hydroxamsäure zur Stabilisierung der Viskosität einer Zusammensetzung die mindestens ein filmbildendes Polymer, das von Polyurethan abgeleitet ist, und ein Verdickungsmittel umfasst, vorzugsweise eine Zusammensetzung nach einem der Ansprüche 1 bis 7.

## Claims

1. Film-forming liquid composition **characterised in that** it comprises:
- at least one film-forming polymer derived from the polyurethane present in the composition with a content of dry matter ranging from 23% to 60% by weight, preferably ranging from 25% to 55% by weight, relative to the total weight of the composition,
- at least one hydroxamic acid, salts and/or complexes thereof and
- at least one thickening agent.

2. Film-forming composition according to claim 1, **characterised in that** the film-forming polymer derived from the polyurethane is known as INCI polyurethane-32.

3. Film-forming composition according to claim 1, **characterised in that** the film-forming polymer derived from the polyurethane consists of a prepolymer
A) of formula: 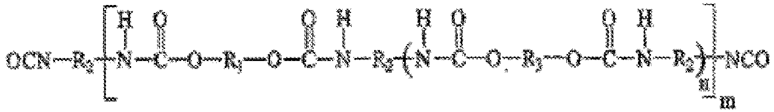 where
R₁ represents a polyhydroxyl radical, preferably dihydroxyl,
R₂ represents a hydrocarbon radical derived from an aliphatic or cycloaliphatic polyisocyanate,
R₃ represents a hydrocarbon radical derived from a diol, potentially with a low molecular weight, optionnally substituted by ionic groups,
n is 0 to 5, and
m is > 1;
B) at least one chain-extending agent according to the formula:
H₂N-R₄-NH₂
where R₄ represents an alkylene or alkylene oxide radical not substituted by ionic or potentially ionic groups; and
C) at least one chain extending agent of formula:
R₅-NH-R₅'-NH₂
where R₅ represents an alkylene radical substituted by ionic groups or potentially ionic groups, and R₅' represents an alkyl radical, in particular C₂-C₄.

4. Film-forming composition according to the preceding claim, **characterised in that** R2 is a mixture of 1,6-hexamethylene diisocyanate and isophorone diisocyanate, R3 is derived from butane-1,4-diol, the chain extender B) is ethylene diamine and the chain extender C) is the sodium salt of N-(2-aminoethyl)-3-aminoethane sulfonic acid.

5. Film-forming composition according to any of the preceding claims, **characterised in that** the hydroxamic acid is present in a content ranging from 0.01 to 10% by weight, preferably from 0.1 to 8%, and more preferably from 0.1 to 5% by weight, relative to the total weight of the composition.

6. Film-forming composition according to any of the preceding claims, **characterised in that** the hydroxamic acid is selected from a group consisting of hexanohydroxamic acid, caprylohydroxamic acid, caprohydroxamic acid, laurohydroxamic acid and mixtures thereof, preferably the hydroxamic acid is caprylohydroxamic acid.

7. Film-forming composition according to any of the preceding claims, **characterised in that** the thickening agent represents 0.01 to 8%, preferably 0.01 to 5%, more preferably 0.01 to 2% by weight relative to the total weight of the composition.

8. Method for obtaining a film from a film-forming composition according to any of claims 1 to 7, **characterised in that**:
i. a composition according to any of claims 1 to 6 is applied onto tissues such as the skin, hair or mucous membranes so as to form a uniform liquid film,
ii. it is allowed to dry for 1 to 5 minutes.

9. Method according to claim 8, **characterised in that** the thickness of the uniform liquid film is greater than 500 nm, preferably between 1 and 300 µm, more preferably between 2 and 200 µm.

10. Film which can be obtained from the method according to any of claims 8 or 9.

11. Use of hydroxamic acid to stabilise the viscosity of a composition comprising at least one film-forming polymer derived from polyurethane and a thickening agent, preferably a composition according to any of claims 1 to 7.
